# EUROPÄISCHE PATENTANMELDUNG

(11) **EP 4 631 634 A1**
(43) Veröffentlichungstag der Anmeldung: **15.10.2025**
(21) Anmeldenummer: 25161723.9
(22) Anmeldetag: 05.03.2025
(51) Int. Cl.: B08B 9/28, B65B 7/28, B65B 55/10, B67C 7/00, B65B 65/00, B65G 47/252, B65B 43/42, B65B 55/02

(54) **DESINFEKTIONSVORRICHTUNG ZUM DESINFIZIEREN VON DOSEN**

(30) Priorität: 25.03.2024 DE 102024108445
(71) Anmelder: KHS GmbH, 44143 Dortmund (DE)
(72) Erfinder: Jakob, Timo, 44143 Dortmund (DE); Seewald-Raider, Alex, 44143 Dortmund (DE); Emrich, Jonas, 44143 Dortmund (DE); Matle, Oliver, 44143 Dortmund (DE)

(57) **Zusammenfassung**

Die Erfindung bezieht sich auf eine Desinfektionsvorrichtung (1) zum Desinfizieren von Dosen (B) mit einem Desinfektionsmittel. Die Desinfektionsvorrichtung (1) weist zumindest eine Fördereinrichtung (2) zum Fördern der Dosen (B) in einer Transportrichtung (TR) ausgehend von einem höheren ersten Höhenniveau (h1) zu einem niedrigeren zweiten Höhenniveau (h2) auf. Die Fördereinrichtung (2) weist zumindest eine geneigte Förderstrecke (3) und ferner wenigstens eine in Transportrichtung (TR) vor oder nach der geneigten Förderstrecke (3) angeordnete erste horizontal verlaufende Förderstrecke (4) auf. Sowohl die mindestens eine erste horizontal verlaufende Förderstrecke (4) als auch die geneigte Förderstrecke (3) bilden jeweils einen Behandlungsabschnitt (10, 10', 20, 20', 30, 40, 50) zur Durchführung von zumindest einem Behandlungsschritt beim Desinfizieren der Dosen (B) aus.

## Beschreibung

Die Erfindung bezieht sich auf eine Desinfektionsvorrichtung zum Desinfizieren von Dosen, insbesondere zum Desinfizieren von Getränkedosen, die zum Befüllen mit einem Getränk vorgesehenen sind. Die Erfindung bezieht sich ferner auf ein Verfahren zum Desinfizieren von Dosen sowie auf eine Behandlungsanlage zum Behandeln von Dosen mit einer derartigen Desinfektionsvorrichtung.

Handelsübliche Getränkedosen stellen weit verbreitete Verpackungen für verschiedene Getränke dar und erfreuen sich großer Beliebtheit bei Verbrauchern, wobei derartige Getränkedosen in unterschiedlichsten Varianten auf dem Markt angeboten werden. Dementsprechend sind auch Vorrichtungen oder Anlagen zum Befüllen von Dosen mit Getränken, nämlich Dosen-Abfüllvorrichtungen bzw. Dosen-Abfüllanlagen, hinreichend bekannt.

Wie grundsätzlich beim Abfüllen flüssiger Nahrungsmittel gilt es auch bei der Dosen-abfüllung bestimmte Hygienemaßstäbe bzw. vorgeschriebene Hygienestandards einzuhalten, um damit die für Nahrungsmittel geltenden hygienischen Voraussetzungen und Kriterien zu erfüllen. Je nach Hygiene-Klassifizierung der abzufüllenden Nahrungsmittel, insbesondere der abzufüllenden Getränke, müssen die Abfüllvorrichtungen bzw. Abfüllanlagen dabei einem bestimmten Niveau an Hygienekriterien genügen.

Zur Einhaltung der entsprechenden Hygienekriterien ist gemäß allgemein geltenden Richtlinien und Regularien beispielsweise die so genannte VDMA-Hygieneklassifizierung zu berücksichtigen. So werden in derzeit bekannten Abfüllanlagen beispielsweise Produkte der VDMA-Hygieneklasse III (pH-Wert < 4,6; CO₂-Gehalt > 4,5 g/l), wie etwa alkoholfreies Radler, in der Regel in eine unentkeimte Dose gefüllt und anschließend pasteurisiert, um den einzuhaltenden Hygienekriterien für solche Produkte der VDMA-Hygieneklasse III zu entsprechen.

Nachteilig ist jedoch die Pasteurisierung der bereits abgefüllten Getränke zeitaufwendig sowie energie- und ressourcenintensiv und verursacht hohe Kosten im Betrieb.

Darüber hinaus erfordert die Integration eines Pasteurs in die Abfüllanlage neben hohen Investitionskosten auch einen erheblichen konstruktiven Aufwand sowie einen großen Platzbedarf in der Aufstellung der Abfüllanlage. Somit besteht weiterhin Bedarf an verbesserten Abfüllanlagen zur hygienisch einwandfreien Dosenabfüllung.

Eine Aufgabe der Erfindung ist es, eine Desinfektionsvorrichtung zum Desinfizieren von Dosen aufzuzeigen, die die Nachteile des Standes der Technik überwindet und die insbesondere eine betriebskostenschonende sowie ressourcenschonende hygienische Dosenabfüllung ermöglicht, um insbesondere den geltenden Hygienestandards zu genügen.

Die vorliegende Erfindung stellt zur Lösung der Aufgabe eine Desinfektionsvorrichtung zum Desinfizieren von Dosen mit einem Desinfektionsmittel zur Verfügung. Die Desinfektionsvorrichtung weist zumindest eine Fördereinrichtung zum Fördern der Dosen in einer Transportrichtung ausgehend von einem höheren ersten Höhenniveau zu einem niedrigeren zweiten Höhenniveau auf. Die Fördereinrichtung umfasst dabei zumindest eine geneigte Förderstrecke und ferner wenigstens eine in Transportrichtung vor oder nach der geneigten Förderstrecke angeordnete erste horizontal verlaufende Förderstrecke. Sowohl die mindestens eine erste horizontal verlaufende Förderstrecke als auch die geneigte Förderstrecke bilden jeweils einen Behandlungsabschnitt zur Durchführung von zumindest einem Behandlungsschritt beim Desinfizieren der Dosen aus.

Unter einer "Desinfektionsvorrichtung" im vorliegenden Sinne wird eine Vorrichtung bzw. eine Einrichtung verstanden, die dazu ausgebildet ist, leere, für die Befüllung mit einem flüssigen Füllgut, insbesondere mit einem Getränk, vorgesehene bzw. bereitgestellte Dosen zumindest innenseitig hygienisch zu reinigen bzw. zu desinfizieren und insbesondere den erreichten Desinfektions- bzw. Hygienestatus aufrechtzuerhalten. Die vorliegende Desinfektionsvorrichtung kann auch dazu ausgebildet sein, die Dosen gegebenenfalls zusätzlich außenseitig hygienisch zu reinigen bzw. zu desinfizieren.

Dosen im vorliegenden Sinn sind insbesondere Getränkedosen, die bevorzugt aus Weißblech oder aus Aluminium hergestellt sind und beispielsweise gängige Dosengrößen, etwa mit einem Fassungsvermögen von 0,25 Liter, 0,33 Liter oder 0,5 Liter, aufweisen.

Der vorliegend verwendete Begriff "hygienisch gereinigt" bedeutet im Verständnis der Erfindung im Wesentlichen, dass die Dosen in einen derartigen Hygienestatus gebracht und in diesem aufrechterhalten werden, dass die geltenden Hygienestandards für die Abfüllung von Getränken eingehalten sind. Die "hygienische Reinigung" ist daher als Keimreduktion mit einem Mindestfaktor zu verstehen, insbesondere als Desinfektion. Die Begriffe "hygienisch gereinigt" und "desinfiziert" können vorliegend auch synonym verwendet werden.

"Behandlungsschritte beim Desinfizieren der Dosen" sind solche Behandlungsschritte, die für die hygienische Reinigung bzw. für die Desinfektion der Dosen erforderlich sind, wobei die "Behandlungsschritte beim Desinfizieren der Dosen" zumindest ausgewählte der folgenden Behandlungsschritte, insbesondere mehrere oder alle der folgenden Behandlungsschritte in Kombination, umfassen, nämlich ein "Auftragen von Desinfektionsmittel", ein "Einwirken (lassen) des Desinfektionsmittels", ein "Trocknen der Dosen und/oder Aktivieren des Desinfektionsmittels", ein "Spülen der Dosen", und/oder ein "Abtropfen (lassen) und/oder Austropfen (lassen) der Dosen". Während der Behandlungsschritte werden die Dosen insbesondere mittels der Fördereinrichtung in Transportrichtung gefördert.

Der Behandlungsschritt "Auftragen von Desinfektionsmittel" kann ein Einsprühen bzw. Aufsprühen oder ein Einblasen bzw. Aufblasen des Desinfektionsmittels in/auf die Dosen umfassen. Der Behandlungsschritt "Einwirken des Desinfektionsmittels" kann beispielsweise eine zeit- und/oder temperaturabhängige Inkubation bzw. eine zeit- und/oder temperatur-kontrollierte Inkubation der mit dem Desinfektionsmittel beaufschlagten Dosen umfassen. Der Behandlungsschritt "Trocknen der Dosen und/oder Aktivieren des Desinfektionsmittels" kann ein Erhitzen der mit dem Desinfektionsmittel beaufschlagten Dosen und/oder eine Zuführung eines Luftstroms, insbesondere eines Heißluftstroms, umfassen. Der Behandlungsschritt "Spülen der Dosen" kann ein Ausspülen der Dosen mit einem sterilen Spülmedium, beispielsweise mit sterilem Wasser oder ein Ausblasen der Dosen mit steriler Luft umfassen.

In einer die vorliegende Desinfektionsvorrichtung umfassenden Dosen-Abfüllanlage kann vorteilhaft der notwendige Desinfektions- bzw. Hygienestatus bis zum tatsächlichen Füllvorgang und über diesen hinaus bis zum hygienischen Verschließen der Dose aufrechterhalten werden. Eine Rekontamination der zumindest innenseitig hygienisch gereinigten bzw. desinfizierten Dose kann damit vermieden werden. Die Desinfektion der Dosen, insbesondere Getränkedosen, erfolgt mit der erfindungsgemäßen Desinfektionsvorrichtung vor dem Befüllen der Dosen.

Mittels der erfindungsgemäßen Desinfektionsvorrichtung kann vorzugsweise eine Keimreduktionsrate mit einer Log-Stufe von mindestens log3 erreicht werden. Dies ist gleichbedeutend damit, dass die Reduktion von Keimen mindestens 99,9% beträgt. Mit dieser erzielten Keimreduktion in den leeren Dosen kann vorteilhaft unter Einhaltung der erforderlichen Hygienestandards ein vor der Abfüllung mittels einer Kurzzeiterhitzungsanlage (KZE) erhitztes Füllgut abgefüllt werden, wobei auf eine anschließende Pasteurisierung der befüllten Dosen verzichtet werden kann.

In anderen Worten ausgedrückt kann durch die vorliegende Desinfektionsvorrichtung ein Pasteur in der Abfüllanlage eingespart werden, da ein vor der Zuführung zur Füllvorrichtung kurzzeiterhitztes Füllgut in die desinfizierten Dosen hygienisch abgefüllt werden kann. Aufgrund der Desinfektion der Dosen mittels der Desinfektionsvorrichtung kann dabei der Hygienestatus ohne nachträgliche Pasteurisierung der befüllten Dosen aufrechterhalten werden.

Mit der Desinfektionsvorrichtung wird vorteilhaft eine Abfüllung sensibler Getränke unter höchsten hygienischen Maßstäben an die mikrobiologische Reinheit ermöglicht, wobei mindestens die Anforderungen einer so genannten "Ultra Clean Abfüllung", insbesondere auch für Produkte der VDMA-Hygieneklasse III, erreicht und eingehalten werden.

Die Fördereinrichtung der erfindungsgemäßen Desinfektionsvorrichtung ist zur Überbrückung einer Höhendifferenz zwischen dem höheren ersten und dem niedrigeren zweiten Höhenniveau ausgebildet und weist hierzu die geneigte Förderstrecke auf, welche im Wesentlichen die Höhenüberbrückung bewirkt. Das höhere erste Höhenniveau bzw. das niedrigere zweite Höhenniveau können vorliegend auch als oberes bzw. unteres Höhenniveau verstanden werden. Alternativ oder synonym dazu können das höhere erste Höhenniveau bzw. das niedrigere zweite Höhenniveau auch als obere bzw. untere Ebene oder als obere bzw. untere Hallenebene einer Aufstellungshalle bezeichnet werden. Die geneigte Förderstrecke ist im Wesentlichen auch als geneigter, nämlich als höhenüberbrückender oder eine Höhendifferenz überwindender Abschnitt oder Teilabschnitt der Desinfektionsvorrichtung zu verstehen.

Die geneigte Förderstrecke, welche insbesondere auch als Schräge oder Schrägstrecke verstanden werden kann, weist dabei eine Neigung gegenüber der Horizontalen auf und verläuft somit in Transportrichtung mit einer vorgegebenen Steigung vom unteren zum oberen Höhenniveau. Die Höhendifferenz zwischen dem unteren und oberen Höhenniveau beträgt vorzugsweise maximal 9 Meter und liegt beispielsweise in einem Bereich zwischen 4 und 8 Metern, insbesondere zwischen 5 und 7 Metern oder zwischen 4,5 und 6 Metern. Die Neigung der geneigten Förderstrecke beträgt beispielsweise 30°.

In der erfindungsgemäßen Desinfektionsvorrichtung werden die Dosen auf dem oberen bzw. höheren ersten Höhenniveau der Desinfektionsvorrichtung zugeführt, beispielsweise mittels eines so genannten Dosenabräumers im Zuge einer Depalettierung. Auf dem unteren bzw. niedrigeren zweiten Höhenniveau werden die Dosen von der Desinfektionsvorrichtung abgegeben und dabei beispielsweise an eine Füllvorrichtung bzw. an eine Füllmaschine übergeben.

Die mindestens eine erste horizontal verlaufende Förderstrecke ist als horizontal verlaufender, nämlich ebener bzw. in einer Ebene verlaufender Abschnitt oder Teilabschnitt der Desinfektionsvorrichtung zu verstehen. Erfindungsgemäß sind beide Abschnitte oder Teilabschnitte der Desinfektionsvorrichtung, nämlich der geneigte und der horizontale Abschnitt oder Teilabschnitt, zur Durchführung von Behandlungsschritten zur Desinfektion der Dosen ausgebildet. Für die desinfektion der Dosen wird dabei nicht nur die geneigte Förderstrecke, sondern überdies auch die horizontale Förderstrecke genutzt. Auf diese Weise wird vorteilhaft erzielt, dass die erforderlichen Zeiten zum Desinfizieren der Dosen, insbesondere die Desinfektionszeiten, eingehalten werden können, insbesondere auch dann, wenn für die geneigte Förderstrecke Parameter wie etwa Höhenunterschiede, Neigungswinkel und dergleichen beibehalten werden, wie sie beispielsweise als feststehende Standardparameter für einen gravitationsbedingten Transport der Dosen gelten und als solche einzuhalten sind. Durch das "Ausnutzen" der horizontalen wie auch der geneigten Förderstrecke wird eine effiziente Desinfektion erreicht.

Hierdurch ergibt sich insbesondere der Vorteil, dass die Desinfektionsvorrichtung bei einer üblichen bzw. herkömmlichen Aufstellung einer Füllvorrichtung zur Dosenabfüllung, beispielsweise in einer Dosenabfüllanlage, bei der die Dosen auf einer oberen Ebene bzw. einem oberen Höhenniveau zugeführt und auf einer unteren Ebene bzw. unterem Höhenniveau an die Füllmaschine übergeben werden, eingesetzt werden kann. Dabei können die für eine effektive Desinfektion erforderlichen DesinfektionsZeiten eingehalten werden und trotzdem die üblichen baulichen und konstruktiven Gegebenheiten, insbesondere hinsichtlich der Höhendifferenz, beibehalten werden.

Gemäß einer bevorzugten Ausführungsvariante der vorliegenden Erfindung ist als Behandlungsabschnitt zumindest ein Beaufschlagungsabschnitt für eine Beaufschlagung der Dosen mit dem Desinfektionsmittel vorgesehen. Der zumindest eine Beaufschlagungsabschnitt ist dabei durch einen Einlaufbereich der geneigten Förderstrecke gebildet und/oder durch die erste horizontal verlaufende Förderstrecke. Die den Beaufschlagungsabschnitt bildende erste horizontal verlaufende Förderstrecke ist dabei vor der geneigten Förderstrecke, insbesondere in Transportrichtung gesehen vor der geneigten Förderstrecke, angeordnet.

Je nach Anwendung und Bedarf kann die Beaufschlagung der Dosen mit dem Desinfektionsmittel dabei insbesondere abschließend auf dem höheren ersten Höhenniveau erfolgen und/oder die Beaufschlagung kann auf dem höheren ersten Höhenniveau beginnen und entlang des sich vom höheren ersten Höhenniveau aus erstreckenden Einlaufbereich fortgesetzt werden. Auch kann die Beaufschlagung lediglich auf dem sich ausgehend vom höheren ersten Höhenniveau erstreckenden Einlaufbereich durchgeführt werden.

In einer bevorzugten Ausführungsvariante kann die erste horizontal verlaufende Förderstrecke nach der geneigten Förderstrecke, insbesondere in Transportrichtung gesehen nach der geneigten Förderstrecke, angeordnet sein. In dieser bevorzugten Ausgestaltung bildet die erste horizontal verlaufende Förderstrecke einen als Abtropf- und/oder Trocknungsabschnitt ausgebildeten Behandlungsabschnitt und stellt damit einen Behandlungsabschnitt zur Fertigstellung bzw. Beendigung des Desinfektionsvorganges dar. Dabei bildet die erste horizontal verlaufende Förderstrecke insbesondere einen Abschnitt oder Teilabschnitt der Desinfektionsvorrichtung, die vorzugsweise auf dem unteren zweiten Höhenniveau angeordnet ist. In dieser Ausgestaltung kann die erste horizontal verlaufende Förderstrecke ferner insbesondere zur Übergabe der desinfizierten Dosen an die Füllmaschine ausgebildet sein oder zum Weiterfördern der desinfizierten Dosen an eine Überführungs- oder Übergabeeinrichtung.

Bevorzugt weist die Fördereinrichtung ferner wenigstens eine in Transportrichtung vor oder nach der geneigten Förderstrecke angeordnete zweite horizontal verlaufende Förderstrecke auf. Die zweite horizontal verlaufende Förderstrecke bildet ebenfalls einen Behandlungsabschnitt zur Durchführung zumindest eines Behandlungsschritts beim Desinfizieren der Dosen aus. Besonders bevorzugt kann dabei der geneigten Förderstrecke in Transportrichtung jeweils eine horizontal verlaufende Förderstrecke vor- und nachgeordnet sein, so dass die geneigte Förderstrecke bezogen auf die Transportrichtung im Wesentlichen von horizontal verlaufende Förderstrecken flankiert ist.

Besondere Vorteile ergeben sich dadurch, dass die geneigte Förderstrecke zumindest abschnittsweise wenigstens einen als Einwirk- und/oder Aktivierungsabschnitt und/oder einen als Spülabschnitt ausgebildeten Behandlungsabschnitt bildet. Es versteht sich, dass diese genannten Behandlungsabschnitte insbesondere dazu ausgebildet sind, die Dosen zu spülen, insbesondere auszuspülen, und/oder das ein- bzw. aufgebrachte Desinfektionsmittel zu aktivieren und einwirken zu lassen und/oder die Dosen, vorzugsweise unter weiterem Einwirken des Desinfektionsmittels zu trocknen.

Bevorzugt ist in dem zumindest einen Beaufschlagungsabschnitt eine Beaufschlagungsvorrichtung zum Beaufschlagen der Dosen mit dem Desinfektionsmittel vorgesehen. Die Beaufschlagungsvorrichtung ist dabei insbesondere bevorzugt eine Sprüh- oder Blasvorrichtung, die insbesondere mit einer oder mehreren Düsen, insbesondere Sprüh- und/oder Blasdüsen ausgestattet sein kann. Die Beaufschlagungsvorrichtung kann auch als Aufbringvorrichtung oder Einbringvorrichtung zum Aufbringen bzw. Einbringen des Desinfektionsmittels auf/in die Dosen verstanden werden und gewährleistet eine effektive und sichere Beaufschlagung der Dosen mit Desinfektionsmittel, welches flüssig oder auch gas- bzw. dampfförmig sein kann.

Je nach Anwendung und Bedarf kann die Desinfektionsvorrichtung dabei besonders vorteilhaft für eine Nassdesinfektion ausgebildet sein, bei welcher ein flüssiges Desinfektionsmittel, wie zum Beispiel Peressigsäure oder Chlordioxid verwendet wird. Hierzu ist die Beaufschlagungsvorrichtung zur Beaufschlagung mit flüssigem Desinfektionsmittel eingerichtet. Additiv oder alternativ kann die Desinfektionsvorrichtung dabei ebenso bevorzugt und vorteilhaft für eine Trockendesinfektion ausgebildet sein, bei welcher ein gas-/dampfförmiges Desinfektionsmittel, wie zum Beispiel H₂O₂, verwendet wird. Hierzu ist die Beaufschlagungsvorrichtung zur Beaufschlagung mit einem gas-/dampfförmigem Desinfektionsmittel eingerichtet.

Gemäß einer besonders bevorzugten Ausführungsvariante ist ferner eine Heizeinrichtung und/oder eine Spüleinrichtung und/oder eine Trocknungseinrichtung vorgesehen. Die Heizeinrichtung und/oder die Spüleinrichtung und/oder die Trocknungseinrichtung sind dabei vorzugsweise im Bereich des wenigstens einen Einwirk- und/oder Aktivierungsabschnitts und/oder Trocknungs- und/oder Aktivierungsabschnitts und/oder im Bereich des Spülabschnitts angeordnet.

Insbesondere ist die vorteilhaft vorgesehene Spüleinrichtung dazu eingerichtet, die Dosen von unten her zu spülen, während sich diese mit ihrer jeweiligen Dosenöffnung nach unten weisend in einer Kopfüberstellung befinden, nämlich "auf dem Kopf stehend" ausgerichtet sind. So kann beispielsweise die Spüleinrichtung derart angeordnet sein, beispielsweise unterhalb der Förderstrecke, dass die Dosen in Kopfüberstellung oberhalb der Spüleinrichtung vorbeigefördert werden und die Spüleinrichtung einen in vertikaler Richtung nach oben gerichteten Spül- oder Spritzstrahl, beispielsweise einen Strahl sterilen Wassers, in die Dosen abgibt und diese ausspritzt. Die Spüleinrichtung kann in diesen Varianten als Spritzung oder Ausspritzvorrichtung verstanden werden.

Bevorzugt bildet die geneigte Förderstrecke einen Rinser, insbesondere einen Gravitationsrinser, besonders bevorzugt einen linearen Gravitationsrinser. Der Rinser bzw. Gravitationsrinser kann dabei in Form eines ein Stangenrinsers oder Vakuumrinsers ausgebildet sein und kann beispielsweise doppelbahnig ausgeführt sein und/oder ein Zweikanal-Rinser sein. In dieser Ausgestaltung kann die vorliegende Desinfektionsvorrichtung besonders einfach in eine Dosenabfüllanlage integriert werden, die baulich und konstruktiv in üblicher Weise konzipiert ist.

Besondere Vorteile ergeben sich bei der vorliegenden Desinfektionsvorrichtung weiterhin dadurch, dass eine Hygiene-Einhausung vorgesehen ist, die einen Reinraum oder Sterilraum für die Behandlung der Dosen begrenzt. Die Hygiene-Einhausung erstreckt sich bevorzugt durchgehend entlang der geneigten und horizontalen Förderstrecken, und definiert dabei einen von einem Doseneinlauf bis zu einer Dosenübergabe durchgehenden Reinraum oder Sterilraum. Dadurch können die Dosen innerhalb des von der Hygiene-Einhausung begrenzten Rein- oder Sterilraums desinfiziert werden und der Desinfektionsstatus kann bis zur Übergabe an eine nachfolgende Füllvorrichtung bzw. Füllmaschine aufrechterhalten werden. Die Hygiene-Einhausung kann vorliegend auch als Isolator bezeichnet werden.

Hierbei ist ferner eine Sterilluftzuführung zur Sterilluftüberlagerung des Reinraums oder Sterilraums vorgesehen, wobei die Sterilluftzuführung insbesondere so angeordnet und ausgebildet ist, dass sie eine Verdrängerströmung bzw. Verdrängungsströmung, insbesondere eine positive Verdrängerströmung bzw. Verdrängungsströmung in dem Rein- bzw. Sterilraum erzeugt, so dass mittels der Sterilluftzuführung die Rein- oder Sterilzone, nämlich die hygienische Zone bzw. Desinfektionszone oder aseptische Zone aufrechterhalten wird.

Vorzugsweise ist in der geneigten Förderstrecke mindestens eine Wendevorrichtung vorgesehen, um die Dosen zwischen einer Normalstellung und einer Überkopfstellung zu wenden. Beispielweise umfasst die Wendevorrichtung mehrere Stangen oder Geländer- bzw. Führungselemente zum Führen der Dosen, wobei die Stangen oder Geländer- bzw. Führungselemente insbesondere in Form einer gewendelten Geländerführung ausgebildet und so konfiguriert sind, dass sie eine Zwangsführung für die Dosen bilden, durch welche die in Transportrichtung bewegten Dosen während ihres Transports bzw. während ihrer Förderung entlang der geneigten Förderstrecke um 180° umgedreht werden. Aufgrund der Zwangsführung entlang der genannten Geländerführung erfahren die Dosen eine Drehung um 180°, so dass sie am Ende der Geländerführung kopfüber in Überkopfstellung bzw. Überkopfposition stehen.

In der "Überkopfstellung", welche auch als Kopfüberstellung bezeichnet werden kann, weisen die zur Befüllung vorgesehenen und deshalb oberseitig offenen Dosen bzw. Leer-Dosen mit ihrer offenen Oberseite, nämlich mit ihrer jeweiligen Dosenöffnung, nach unten und stehen quasi auf dem Kopf. In der "Normalstellung" weist die offene Oberseite der Dose nach oben, das heißt die Dosen sind mit ihrem Bodenabschnitt nach unten weisend ausgerichtet und befinden sich damit in derselben Orientierung, die sie auch während der Befüllung einnehmen.

Beispielsweise laufen die Dosen in Normalstellung in die geneigte Förderstrecke ein und werden mittels der die Zwangsführung bildenden Wendevorrichtung während der Bewegung entlang der geneigten Förderstrecke in die Überkopfstellung gedreht. Besonders bevorzugt werden die Dosen während ihrer Bewegung über die geneigte Förderstrecke schließlich auch wieder in Normalstellung zurückgedreht, bevor sie die Desinfektionsvorrichtung verlassen und beispielsweise an eine Füllmaschine übergeben werden. Auch das "Rückdrehen" von der Überkopfstellung in die Normalstellung kann zum Beispiel durch eine, wiederum eine Zwangsführung bewirkende, gewendelte Geländerführung sichergestellt werden.

Auch ist es denkbar, dass in alternativen Ausführungsformen Wendeeinrichtungen in Form von Greifern oder dergleichen vorgesehen sind, die flankierend zu der geneigten Förderstrecke angeordnet sind und welche die Dosen im Bereich ihres Einlaufens in die geneigte Förderstrecke in Überkopfstellung drehen und im Bereich ihres Auslaufens aus der geneigten Förderstrecke wieder in die Normalstellung zurückdrehen. Für derartige Wendeeinrichtungen in Form von Greifern oder dergleichen ist es insbesondere auch denkbar, dass diese im Bereich der vor und nach der geneigten Förderstrecke angeordneten horizontal verlaufenden Förderstrecken angeordnet sein können.

Alternativ oder zusätzlich kann auch vorgesehen sein, dass eine Wendevorrichtung und/oder eine Wendeeinrichtung, insbesondere ein Dosenwender, im Bereich der ersten horizontal verlaufenden Förderstrecke vorgesehen ist, insbesondere stromaufwärts des Beaufschlagungsabschnittes, so dass die Dosen beispielsweise bereits in einer Überkopfstellung in den Beaufschlagungsabschnitt einlaufen und in dieser Überkopfstellung von unten her mit Desinfektionsmittel beaufschlagt werden können. Die vorliegende Erfindung umfasst auch eine Behandlungsanlage zum Behandeln von Dosen, wobei die Behandlungsanlage eine Desinfektionsvorrichtung aufweist, wie sie voranstehend beschrieben ist. Ferner weist die Behandlungsanlage eine der Desinfektionsvorrichtung nachgeschaltete Füllvorrichtung zum Befüllen der Dosen mit einem flüssigen Füllgut sowie eine der Füllvorrichtung nachgeschaltete und/oder mit der Füllvorrichtung verblockte Schließvorrichtung zum Verschließen der befüllten Dosen auf.

Das flüssige Füllgut zur Abfüllung in die Dosen ist vorzugsweise ein Getränk. Insbesondere kann es sich dabei um Wasser, um alkoholhaltiges oder alkoholfreies Bier, um alkoholhaltiges oder alkoholfreies Radler, um ein alkoholhaltiges oder alkoholfreies Biermischgetränk, um ein Malzgetränk, um alkoholhaltige oder alkoholfreie Getränke auf Basis von Wein, um kohlensäurehaltige Softgetränke, um Spritzer oder dergleichen handeln.

Die Füllvorrichtung ist bevorzugt eine Füllvorrichtung umlaufender Bauart, das heißt eine Rundläufer-Füllmaschine, mit einem antreibbaren, um eine vertikale Maschinenachse umlaufend rotierenden Rotor mit einer Vielzahl an Füllpositionen zum Befüllen der Dosen. Jede Füllposition ist über entsprechende Füllgutleitungen, und dabei steuerbar über entsprechend vorgesehene Ventileinrichtungen und Füllventile, fluidisch an einen vorzugsweise ringförmig ausgebildeten Füllgutkessel angeschlossen. In bekannter Weise steht der Füllgutkessel über eine Füllgutzufuhr mit einem Vorrat oder Tank für das flüssige Füllgut in Verbindung.

Auch die Schließvorrichtung ist bevorzugt eine als Rundläufer-Verschließer ausgebildete Schließvorrichtung umlaufender Bauart.

Vorzugsweise umfasst die Schließvorrichtung eine Dosendeckelzuführung mit einer Dosendeckeldesinfektionsvorrichtung. Auf diese Weise wird ein hygienisch einwandfreies Befüllen inklusive eines hygienisch einwandfreien Verschließens der Dosen sichergestellt.

Gemäß einer bevorzugten Ausführungsvariante der Behandlungsanlage umfasst diese eine mit der Füllvorrichtung verbundene Kurzzeiterhitzungsanlage zum Sterilisieren des Füllguts vor dessen Zuführung zu der Füllvorrichtung.

Bevorzugt weist die Behandlungsanlage eine Hygiene-Einhausung auf, in welcher die Füllvorrichtung aufgenommen ist und die unmittelbar an die Desinfektionsvorrichtung anschließt. Über entsprechende Hygieneschleusen und/oder Hygienebarrieren kann dabei sichergestellt werden, dass die desinfizierten Dosen unter Aufrechterhaltung des Desinfektionsstatus von der Desinfektionsvorrichtung an die Füllvorrichtung übergeben werden können.

Beispielsweise kann dabei die Hygiene-Einhausung der Desinfektionsvorrichtung unmittelbar an die Hygiene-Einhausung der Füllvorrichtung anschließen oder in diese übergehen. In jedem Fall ist insbesondere der durch die Hygiene-Einhausung der Desinfektionsvorrichtung definierte Sterilraum oder Reinraum mit einem Rein-/Steril- oder Aseptikraum innerhalb der Hygiene-Einhausung der Füllvorrichtung als ein mindestens strömungstechnisch verbundener durchgehender Rein-/Steril- oder Aseptikraum anzusehen.

In ähnlicher Weise ist bevorzugt auch die Schließvorrichtung in einer Hygiene-Einhausung aufgenommen, wobei die Schließvorrichtung in einer eigenen Hygiene-Einhausung angeordnet sein kann oder alternativ ebenfalls in der dann eine gemeinsame Hygiene-Einhausung bildenden Hygiene-Einhausung der Füllvorrichtung. Besonders bevorzugt ist der Schließvorrichtung ohne Zwischenschaltung einer Pasteurisationsanlage eine Dosen-Verpackungsmaschine und/oder Dosen-Palettiermaschine nachgeschaltet. Darunter ist insbesondere zu verstehen, dass die Behandlungsanlage unter Verzicht auf ein Pasteurisieren nach dem Füllen der Dosen betrieben wird, das heißt, dass die Behandlungsanlage unter Verzicht auf einen stromabwärts der Füllvorrichtung angeordneten Pasteur, insbesondere Tunnel-Pasteur, konfiguriert ist.

Die vorliegende Erfindung betrifft auch ein Verfahren zum Desinfizieren von Dosen mit einem Desinfektionsmittel, bei dem die Dosen mittels einer Fördereinrichtung in einer Transportrichtung ausgehend von einem höheren ersten Höhenniveau zu einem niedrigeren zweiten Höhenniveau entlang zumindest einer geneigten Förderstrecke sowie entlang zumindest einer vor oder nach der geneigten Förderstrecke angeordneten ersten horizontal verlaufenden Förderstrecke gefördert werden, wobei die Dosen bei dem Verfahren sowohl in einem durch die erste horizontal verlaufende Förderstrecke gebildeten Behandlungsabschnitt als auch in einem durch die geneigte Förderstrecke gebildeten Behandlungsabschnitt mit jeweils zumindest einem Behandlungsschritt zum Desinfizieren behandelt werden.

Für das vorliegende Verfahren wird auf die im Zusammenhang mit der Desinfektionsvorrichtung bereits beschriebenen Besonderheiten und Vorteile der Erfindung verwiesen, welche gleichermaßen auch für das Verfahren zum Desinfizieren von Dosen zutreffen.

Die Erfindung wird im Folgenden anhand der Figuren an Ausführungsbeispielen näher erläutert. Es zeigen:
- Fig. 1: eine grob schematisch skizzierte und stark vereinfacht dargestellte Ansicht einer Ausführungsform der Desinfektionsvorrichtung;
- Fig. 2: eine grob schematisch skizzierte und stark vereinfacht dargestellte Ansicht einer weiteren Ausführungsform der Desinfektionsvorrichtung;
- Fig. 3: eine grob schematisch skizzierte und stark vereinfacht dargestellte Ansicht einer noch weiteren Ausführungsform der Desinfektionsvorrichtung und
- Fig. 4: stark vereinfacht und grob schematisch skizziert eine Behandlungsanlage in Draufsicht.

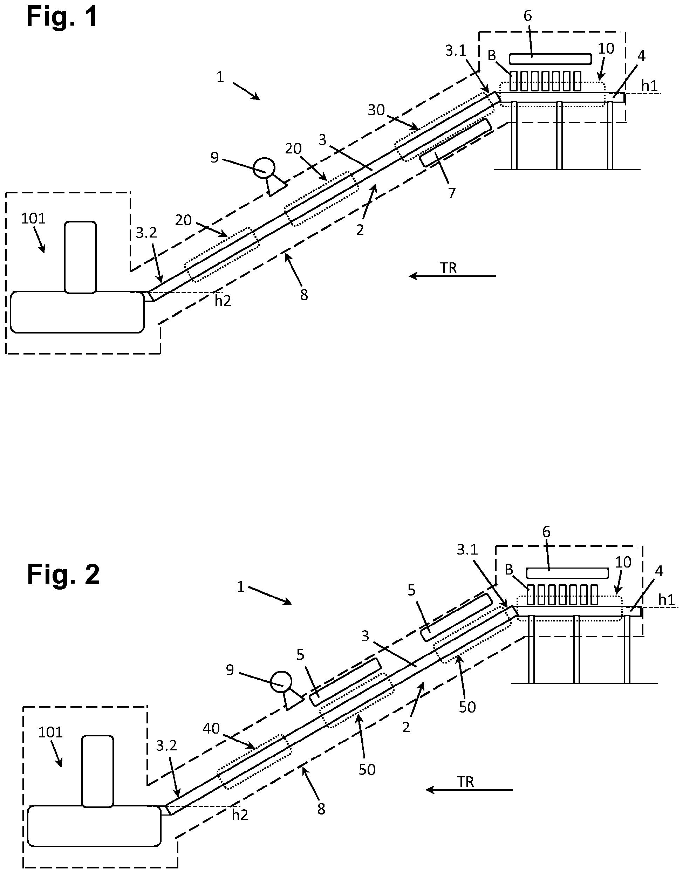

Die Figuren 1 bis 3 zeigen jeweils anhand einer grob schematisch skizzierten und stark vereinfacht dargestellten Ansicht verschiedene Ausführungsformen der Desinfektionsvorrichtung 1 zum Desinfizieren von Dosen B mit einem Desinfektionsmittel. Die Desinfektionsvorrichtung 1 kann bei der Dosen-Abfüllung verwendet werden, um leere, zum Befüllen mit einem Getränk bereitgestellte Dosen B wenigstens innenseitig zu desinfizieren, so dass mindestens die hygienischen Standards für eine "Ultra Clean Abfüllung" bei der Abfüllung von Getränken erfüllt sind und die entsprechenden, hohen Maßstäbe an die mikrobiologische Reinheit erreicht und eingehalten werden können.

Generell weist die vorliegende Desinfektionsvorrichtung 1 eine Fördereinrichtung 2 zum Fördern der Dosen B in einer Transportrichtung TR auf, wobei die Transportrichtung TR im Wesentlichen auch einer Prozessrichtung oder Behandlungsrichtung entspricht, in welcher die Dosen B in aufeinanderfolgenden Behandlungsschritten zum Desinfizieren behandelt, das heißt desinfiziert werden.

Die Fördereinrichtung 2 weist eine geneigte Förderstrecke 3 sowie ferner eine erste horizontal verlaufende Förderstrecke 4 auf. Die geneigte Förderstrecke 3 erstreckt sich mit einer Neigung, in den dargestellten Beispielen mit einer Neigung von etwa 30°, gegenüber der Horizontalen und verläuft ausgehend von einem höheren ersten Höhenniveau h1 zu einem niedrigeren zweiten Höhenniveau h2 bzw. fällt von dem oberen Höhenniveau h1 zu dem unteren Höhenniveau h2 hin ab. Auf diese Weise überbrückt bzw. überwindet die geneigte Förderstrecke 3 eine Höhendifferenz zwischen dem höheren ersten Höhenniveau h1 und dem niedrigeren zweiten Höhenniveau h2. Das obere bzw. höhere erste Höhenniveau h1 und das untere bzw. niedrigere zweite Höhenniveau h2 definieren im Wesentlichen eine obere und untere Ebene einer Aufstellungshalle, in der die Desinfektionsvorrichtung 1 aufgestellt ist, so dass die geneigte Förderstrecke 3 eine Höhendifferenz zwischen der oberen und unteren Ebene der Aufstellungshalle überbrückt bzw. überwindet. Dadurch können die Dosen B auf der oberen Ebene der Desinfektionsvorrichtung 1 zugeführt und auf der unteren Ebene aus dieser wieder abgeführt werden.

Bei allen Ausführungsvarianten der vorliegenden Desinfektionsvorrichtung 1 bildet sowohl die erste horizontal verlaufende Förderstrecke 4 als auch die geneigte Förderstrecke 3 jeweils einen Behandlungsabschnitt 10, 10', 20, 20', 30, 40, 50 zur Durchführung von zumindest einem Behandlungsschritt beim Desinfizieren der Dosen B.

Im Beispiel der Figur 1 ist die erste horizontal verlaufende Förderstrecke 4 in Transportrichtung TR vor der geneigten Förderstrecke 3 angeordnet und verläuft damit auf dem der oberen Ebene entsprechenden höheren ersten Höhenniveau h1. In der gezeigten Ausführungsvariante bildet die erste horizontal verlaufende Förderstrecke 4 einen für eine Beaufschlagung der Dosen B mit dem Desinfektionsmittel vorgesehenen und ausgebildeten Beaufschlagungsabschnitt 10.

Dem Beaufschlagungsabschnitt 10 zugeordnet ist dabei eine Beaufschlagungsvorrichtung 6 zum Beaufschlagen der Dosen B mit dem Desinfektionsmittel. Mit der Beaufschlagungsvorrichtung 6, welche im Beispiel der Figur 1 als Sprühvorrichtung ausgebildet ist, kann das Desinfektionsmittel zumindest innen in die Dosen eingesprüht werden. Die Sprühvorrichtung 6 ist dazu beispielsweise mit einer oder mehreren Sprühdüsen ausgestattet.

In dem in Figur 1 dargestellten Beispiel ist die Desinfektionsvorrichtung 1 insbesondere angepasst und ausgelegt für eine Nassdesinfektion, bei der innerhalb des Beaufschlagungsabschnitts 10 mittels der Beaufschlagungsvorrichtung 6 ein flüssiges Desinfektionsmittel, wie zum Beispiel Peressigsäure oder Chlordioxid, aufgesprüht bzw. in die Dosen B eingesprüht wird. Die den Beaufschlagungsabschnitt 10 bereitstellende horizontale Förderstrecke 4, nämlich der horizontale Abschnitt oder Teilabschnitt der Desinfektionsvorrichtung 1, bildet hier einen einlaufenden oder anfänglichen Behandlungsabschnitt 10 zur Durchführung eines frühen oder anfänglichen bzw. eines initialen oder ersten Behandlungsschrittes beim Desinfizieren der Dosen B.

Im Beispiel der Figur 1 sind die Dosen B auf der horizontalen Förderstrecke 4, nämlich im Beaufschlagungsabschnitt 10, in Normalstellung mit nach oben weisenden oberseitigen Dosenöffnungen orientiert, das heißt, die Dosen B sind aufrechtstehend in einer Orientierung "richtig herum" ausgerichtet. Die Beaufschlagung der Dosen B mit Desinfektionsmittel, nämlich das Einsprühen des Desinfektionsmittels über die Beaufschlagungsvorrichtung 6 erfolgt dabei von oben her, indem die Dosen B unter der oberhalb der horizontalen Förderstrecke 4 angeordneten Beaufschlagungsvorrichtung 6 durchgeführt werden und die Beaufschlagungsvorrichtung 6 die Dosen von oben her mit Desinfektionsmittel einsprüht.

Bei dem Beispiel der Figur 1 erfahren die Dosen in einem Einlaufbereich 3.1 der geneigten Förderstrecke 3 auf ihrem Bewegungsweg beim Weitertransport in Transportrichtung TR eine Drehung und werden von der Normalstellung in eine Überkopfstellung umgedreht, in welcher die oberseitigen Dosenöffnungen nach unten weisen und die leeren Dosen somit auf dem Kopf stehen.

Das Umdrehen der Dosen B von der Normalstellung in die Überkopfstellung kann auch als Dosenwenden bezeichnet werden und erfolgt beispielsweise mittels einer Wendevorrichtung, die vorzugsweise eine aus mehreren Stangen oder Geländer- bzw. Führungselementen gebildete gewendelte Geländerführung umfasst, welche eine Zwangsführung für die bewegten Dosen B bewirkt, so dass diese während ihres Transports um nahezu 180° oder um 180° umgedreht und in Überkopfstellung gebracht werden.

Im Einlaufbereich 3.1 und/oder anschließend an den Einlaufbereich 3.1 bildet die geneigte Förderstrecke 3 einen als Spülabschnitt 30 ausgebildeten Behandlungsabschnitt, in dem die über die geneigte Förderstrecke 3 bewegten, in Kopfüberstellung bzw. Überkopfstellung befindlichen, das heißt, auf dem Kopf stehenden, Dosen B von unten her mit einem sterilen Spülmedium, beispielsweise mit sterilem Wasser, gespült werden können. Hierfür ist bei der Desinfektionsvorrichtung 1 eine zumindest teilweise unterhalb der geneigten Förderstrecke 3 angeordnete Spüleinrichtung 7 vorgesehen, oberhalb derer die Dosen B vorbeigeführt werden. Die Spüleinrichtung 7, welche auch als Spritzung oder Ausspritzvorrichtung verstanden werden kann, gibt dabei einen in vertikaler Richtung nach oben gerichteten Spül- oder Spritzstrahl in die Dosen ab und spritzt diese aus.

Dem Spülabschnitt 30 in Transportrichtung TR nachgeordnet bildet die geneigte Förderstrecke 3 zumindest einen (in der Darstellung des Beispiels der Figur 1 zwei) als Abtropf- und/oder Trocknungsabschnitt 20 ausgebildeten Behandlungsabschnitt aus, in dem das Spülmedium, insbesondere das sterile Wasser, aus den über die geneigte Förderstrecke 3 bewegten und immer noch in Kopfüberstellung befindlichen Dosen B austropfen kann. Nach dem vollständigen Austropfen, welches einem Austrocknen oder Trocknen der Dosen B gleichkommt, sind die Dosen B desinfiziert und stehen im Wesentlichen für die Weiterverarbeitung bzw. Weiterbehandlung, insbesondere für die hygienische Befüllung bereit.

Bevor die desinfizierten Dosen B aus der Desinfektionsvorrichtung 1 abgegeben bzw. ausgeschleust und beispielsweise an eine stromabwärts der Desinfektionsvorrichtung 1 angeordnete Füllvorrichtung 101 übergeben werden, erfolgt im Beispiel der Figur 1 ein erneutes Dosenwenden, um die Dosen B von der Kopfüberstellung wieder in die Normalstellung zurückzudrehen. Dieses Dosenwenden wird beispielsweise in einem Auslaufbereich 3.2 der geneigten Förderstrecke 3 durchgeführt, beispielsweise ebenfalls mittels derselben oder einer weiteren Wendevorrichtung, die beispielsweise wiederum eine aus mehreren Stangen oder Geländer- bzw. Führungselementen gebildete gewendelte Geländerführung umfassen kann, welche eine Zwangsführung für die bewegten Dosen B bewirkt, so dass diese während ihres Transports um 180° umgedreht und zurück in Normalstellung gebracht werden.

In einer in den Figuren zwar nicht bildlich dargestellten, jedoch ebenso denkbaren und bevorzugten Ausführungsvariante, welche von ihrem grundsätzlichen Aufbau her bzw. in ihrer grundsätzlichen Konfiguration sehr ähnlich bzw. analog zu dem Beispiel der Figur 1 ausgebildet ist, kann alternativ auch vorgesehen sein, die Dosen B bereits stromaufwärts des Beaufschlagungsabschnittes 10, das heißt im Bereich der horizontalen Förderstrecke 4 und dabei in Transportrichtung TR vor dem Beaufschlagungsabschnitt 10, zu wenden und in Überkopfstellung zu bringen.

In einer derartigen Ausführungsform ist - im Unterschied zu dem in Figur 1 dargestellten Beispiel - im Bereich der horizontalen Förderstrecke 4 vor dem Beaufschlagungsabschnitt 10 ein Dosenwender vorgesehen, der die Dosen B auf den Kopf dreht, so dass diese im Beaufschlagungsabschnitt 10 mit nach unten weisenden Dosenöffnungen auf der horizontalen Förderstrecke 4 bereitstehen. Die Beaufschlagungsvorrichtung 6 ist in dieser denkbaren Variante unterhalb der Fördereinrichtung 2 angeordnet, so dass die Beaufschlagung mit Desinfektionsmittel von unten her in die bereits gewendeten und in Überkopfstellung befindlichen Dosen B erfolgt, welche oberhalb der Beaufschlagungsvorrichtung 6 vorbeigeführt werden. Die Dosen B verbleiben dann bei dem in Transportrichtung TR erfolgenden Weitertransport in den Einlaufbereich 3.1 der geneigten Förderstrecke 3 (insbesondere unter Verzicht auf ein erneutes Wenden bzw. Drehen der Dosen B im Einlaufbereich 3.1) in Überkopfstellung und können vorzugsweise weiter behandelt werden, wie oberhalb für das Beispiel der Figur 1 beschrieben wurde.

In den Figuren 1 bis 3 mit gestrichelter Linie schematisch angedeutet und mit dem Bezugszeichen 8 gekennzeichnet, ist bei der Desinfektionsvorrichtung 1 gemäß den gezeigten Beispielen ferner eine Hygiene-Einhausung 8 vorgesehen, die einen Reinraum oder Sterilraum für die Behandlung der Dosen B begrenzt. Die Hygiene-Einhausung 8 erstreckt sich dabei mindestens über die gesamte Fördereinrichtung 2 und ist derart durchgehend bzw. durchgängig ausgebildet, dass die Dosen B über die gesamte Dauer und den gesamten Weg des Durchlaufens der Desinfektionsvorrichtung 1 innerhalb des Reinraums oder Sterilraums aufgenommen sind. Die Hygiene-Einhausung 8 geht im Beispiel der Figuren 1 bis 3 in eine Hygiene-Einhausung der Füllvorrichtung 101 über, so dass der Reinraum bzw. Sterilraum mit einem Aseptikraum der Füllvorrichtung 101 durchgehend ausgebildet ist und diese gemeinsam einen durchgehenden Sterilraum bzw. Isolatorraum bilden.

Um die erforderlichen Sterilraum-Bedingungen innerhalb der Hygiene-Einhausung 8 aufrechtzuerhalten, ist bei der dargestellten Desinfektionsvorrichtung 1 eine Sterilluftzuführung 9 zur Sterilluftüberlagerung des Reinraums oder Sterilraums vorgesehen. Die Sterilluftzuführung 9 ist dabei so angeordnet, dass eine positive Verdrängerströmung erzeugt werden kann. Beispielsweise ist die Sterilluftzuführung 9 hierzu im Bereich der geneigten Förderstrecke 3 angeordnet und derart an die Hygiene-Einhausung 8 angeschlossen, dass die Sterilluft dort vermittels der Sterilluftzuführung 9 einströmt, wobei ein Ausströmen von Luft an einem auf dem höheren ersten Höhenniveau h1 befindlichen Ende der Hygiene-Einhausung 8 erlaubt wird, welches im Beispiel der Figur 1 mit dem einlaufenden Ende der ersten horizontal verlaufenden Förderstrecke 4 zusammenfällt.

Die Figur 2 zeigt ein alternatives Ausführungsbeispiel der Desinfektionsvorrichtung 1, welches insbesondere für eine Trockendesinfektion angepasst und ausgelegt ist. Unterschiedlich zu dem Beispiel der Figur 1 befinden sich die Dosen B bei der Ausführungsform gemäß Figur 2 auf ihrem gesamten Weg durch die Desinfektionsvorrichtung 1 in Normalstellung und sind stets mit nach oben weisenden oberseitigen Dosenöffnungen orientiert. Wie im Ausführungsbeispiel der Figur 1 erfolgt die Beaufschlagung der Dosen B mit dem Desinfektionsmittel, nämlich das Einsprühen des Desinfektionsmittels über die Beaufschlagungsvorrichtung 6 von oben her in dem durch die erste horizontale Förderstrecke 4 gebildeten Beaufschlagungsabschnitt 10. Ein Wenden der Dosen entfällt in dieser Ausführungsvariante der Figur 2.

In Transportrichtung TR gesehen an den Beaufschlagungsabschnitt 10 anschließend bildet die geneigte Förderstrecke 3 in ihrem Einlaufbereich 3.1 Behandlungsabschnitte, die insbesondere als Einwirk- und/oder Aktivierungsabschnitte 40 bzw. als Trocknungs- und/oder Aktivierungsabschnitte 50 ausgebildet sind. In den Trocknungs- und/oder Aktivierungsabschnitten 50 erfolgt eine Trocknung und/oder Aktivierung des aufgebrachten Desinfektionsmittels, beispielsweise eine Hitzeaktivierung, wobei hierfür eine Heizeinrichtung 5 zum Erhitzen der Dosen B vorgesehen ist. Die Heizeinrichtung 5 ist im Beispiel der Figur 2 als ein oberhalb der Fördereinrichtung 2 angeordnetes Gebläse, insbesondere als Heißluftgebläse ausgebildet, welches heiße Luft von oben her in die Dosen B einblasen und gegebenenfalls auch außenseitig auf die Dosen B aufblasen kann. Beispielhaft folgen in Figur 2 zwei Trocknungs- und/oder Aktivierungsabschnitte 50 aufeinander, von denen jeder mit einem Heißluftgebläse 5 ausgestattet ist.

Den Trocknungs- und/oder Aktivierungsabschnitten 50 in Transportrichtung TR nachgeordnet folgt ein weiterer Einwirk- und/oder Aktivierungsabschnitt 40, in dem das Einwirken bzw. Trocknen fertiggestellt und dadurch die Desinfektion abgeschlossen wird, um die desinfizierten Dosen B wiederum für die Übergabe an die Füllvorrichtung 101 bereitzustellen.

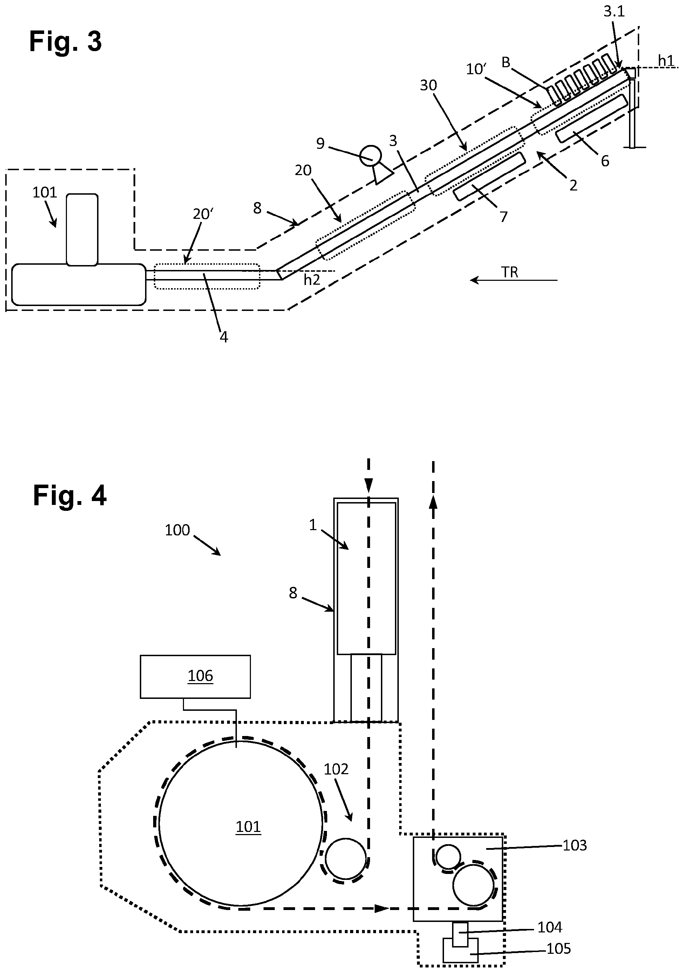

Die Figur 3 zeigt ein weiteres alternatives Ausführungsbeispiel der Desinfektionsvorrichtung 1, welches insbesondere für eine Nassdesinfektion angepasst und ausgelegt ist und bei der die erste horizontal verlaufende Förderstrecke 4 in Transportrichtung TR nach der geneigten Förderstrecke 3 angeordnet ist und damit auf dem der unteren Ebene entsprechenden niedrigeren zweiten Höhenniveau h2 verläuft.

In dieser Ausführungsvariante bildet der Einlaufbereich 3.1 der geneigten Förderstrecke 3 den Beaufschlagungsabschnitt 10', in dem das flüssige Desinfektionsmittel mittels der Beaufschlagungsvorrichtung 6 aufgesprüht bzw. in die Dosen B eingesprüht wird. Die Beaufschlagungsvorrichtung 6 ist hierbei im Einlaufbereich 3.1 vorgesehen und unterhalb der Fördereinrichtung 2 angeordnet, so dass die Beaufschlagung mit Desinfektionsmittel von unten her erfolgt, und zwar in die bereits gewendeten und in Überkopfstellung befindlichen Dosen B, welche oberhalb der Beaufschlagungsvorrichtung 6 vorbeigeführt werden. Die Dosen B bleiben zunächst auf ihrem Bewegungsweg beim Weitertransport in Transportrichtung TR in Überkopfstellung.

Weiter in Transportrichtung TR bildet die geneigte Förderstrecke 3 den Spülabschnitt 30, in dem die über die geneigte Förderstrecke 3 bewegten, in Kopfüberstellung befindlichen Dosen B von unten her mit einem Spülmedium, beispielsweise mit sterilem Wasser mittels der zumindest teilweise unterhalb der geneigten Förderstrecke 3 angeordneten Spüleinrichtung 7 gespült werden. Dem Spülabschnitt 30 in Transportrichtung TR nachgeordnet bildet die geneigte Förderstrecke 3 einen ersten Abtropf- und/oder Trocknungsabschnitt 20.

Die in Transportrichtung TR gesehen nach der geneigten Förderstrecke 3 angeordnete erste horizontal verlaufende Förderstrecke 4 bildet einen in der Waagerechten bzw. in der Horizontalen orientierten Abtropf- und/oder Trocknungsabschnitt 20', über den sichergestellt wird, dass die Dosen B vollständig austropfen bzw. trocknen können und fertig desinfiziert für die Weiterverarbeitung bzw. Weiterbehandlung, insbesondere für die hygienische Befüllung bereitstehen.

Bevor die desinfizierten Dosen B aus der Desinfektionsvorrichtung 1 abgegeben bzw. ausgeschleust und beispielsweise an die Füllvorrichtung 101 übergeben werden, erfolgt im Beispiel der Figur 3 ein Dosenwenden, um die Dosen B von der Kopfüberstellung in die Normalstellung zu drehen. Dieses Dosenwenden kann hier in einem (in der Figur nicht näher bezeichneten) Auslaufbereich der ersten horizontal verlaufenden Förderstrecke 4 durchgeführt werden.

Figur 4 zeigt stark vereinfacht und nur grob schematisch skizziert eine Behandlungsanlage 100 zum Behandeln von Dosen B in einer Draufsicht. Die dargestellte Behandlungsanlage 100 weist eine wie vorliegend beschriebene Desinfektionsvorrichtung 1 auf. Der Desinfektionsvorrichtung 1 nachgeschaltet ist eine Füllvorrichtung 101 vorgesehen zum Befüllen der desinfizierten Dosen B mit einem flüssigen Füllgut, wobei die desinfizierten Dosen B nach dem Desinfizieren der Füllvorrichtung 101 zugeführt werden.

Ein Behandlungspfad oder Behandlungsweg, auf dem die Dosen B während der Behandlung durch die Behandlungsanlage 100 gefördert werden, ist in der Figur 4 anhand gestrichelter Linien angedeutet, die mit Pfeilen versehen sind.

Die Übergabe der desinfizierten Dosen B kann im Wesentlichen unmittelbar von der Desinfektionsvorrichtung 1 zu der Füllvorrichtung 101 erfolgen oder unter Zwischenschaltung einer Übergabeeinrichtung 102, welche hier nicht näher spezifiziert wird und die auch als Transfer- oder Überführungsvorrichtung verstanden werden kann. Der Füllvorrichtung 101 nachgeschaltet und/oder mit der Füllvorrichtung 101 verblockt ist bei der Behandlungsanlage 100 eine Schließvorrichtung 103 zum hygienischen, insbesondere sterilen Verschließen der befüllten Dosen B vorgesehen. Der Schließvorrichtung 103 ist dabei eine Dosendeckelzuführung 104 mit Dosendeckeldesinfektionsvorrichtung 105 zugeordnet, über welche die Schließvorrichtung 103 mit desinfizierten Dosendeckeln versorgt wird.

Wie aus der Figur 4 hervorgeht, kann die Behandlungsanlage 100 eine mit der Füllvorrichtung 101 verbundene Kurzzeiterhitzungsanlage 106 zum Sterilisieren des Füllguts vor dessen Zuführung zu der Füllvorrichtung 101 aufweisen.

Bei der Behandlungsanlage 100 ist eine - in Figur 4 punktiert dargestellte - Hygiene-Einhausung vorgesehen, die einen Isolator bildet bzw. einen Isolatorraum definiert und in welcher die Füllvorrichtung 101 aufgenommen ist. Im dargestellten Beispiel schließt diese Hygiene-Einhausung unmittelbar an die Desinfektionsvorrichtung 1, insbesondere an die Hygiene-Einhausung 8 der Desinfektionsvorrichtung 1 an, so dass die desinfizierten Dosen B im sterilen Hygieneraum rekontaminationsfrei von der Desinfektionsvorrichtung 1 bis zur Füllvorrichtung 101 transportiert werden können. Auch die Schließvorrichtung 103 ist innerhalb der Hygiene-Einhausung angeordnet, so dass erst die vollständig und hygienisch verschlossenen Dosen B den sterilen Hygieneraum bzw. Isolatorraum verlassen

Nach der Schließvorrichtung 103 werden die befüllten, verschlossenen Dosen B ausgeschleust und ohne Zwischenschaltung eines Pasteurs oder einer Pasteurisierungsvorrichtung bzw. einer Pasteurisationsanlage für die Verpackung und/oder Abtransport freigegeben und hierzu beispielsweise einer (in den Figuren nicht dargestellten) Dosen-Verpackungsmaschine und/oder Dosen-Palettiermaschine zugeführt.

### Bezugszeichenliste

- 1: Desinfektionsvorrichtung
- 2: Fördereinrichtung
- 3: geneigte Förderstrecke
- 3.1: Einlaufbereich
- 3.2: Auslaufbereich
- 4: erste horizontal verlaufende Förderstrecke
- 5: Heizeinrichtung
- 6: Beaufschlagungsvorrichtung
- 7: Spüleinrichtung
- 8: Hygiene-Einhausung
- 9: Sterilluftzuführung
- 10, 10': Beaufschlagungsabschnitt
- 20, 20': Abtropf- und/oder Trocknungsabschnitt
- 30: Spülabschnitt
- 40: Einwirk- und/oder Aktivierungsabschnitt
- 50: Trocknungs- und/oder Aktivierungsabschnitt

- 100: Behandlungsanlage
- 101: Füllvorrichtung
- 102: Übergabeeinrichtung
- 103: Schließvorrichtung
- 104: Dosendeckelzuführung
- 105: Dosendeckeldesinfektionsvorrichtung
- 106: Kurzzeiterhitzungsanlage

- B: Dose
- h1: höheres erstes Höhenniveau
- h2: niedrigeres zweites Höhenniveau
- TR: Transportrichtung

## Patentansprüche

1. Desinfektionsvorrichtung (1) zum Desinfizieren von Dosen (B) mit einem Desinfektionsmittel, aufweisend zumindest eine Fördereinrichtung (2) zum Fördern der Dosen (B) in einer Transportrichtung (TR) ausgehend von einem höheren ersten Höhenniveau (h1) zu einem niedrigeren zweiten Höhenniveau (h2), wobei die Fördereinrichtung (2) zumindest eine geneigte Förderstrecke (3) und ferner wenigstens eine in Transportrichtung (TR) vor oder nach der geneigten Förderstrecke (3) angeordnete erste horizontal verlaufende Förderstrecke (4) aufweist, wobei sowohl die mindestens eine erste horizontal verlaufende Förderstrecke (4) als auch die geneigte Förderstrecke (3) jeweils einen Behandlungsabschnitt (10, 10', 20, 20', 30, 40, 50) zur Durchführung von zumindest einem Behandlungsschritt beim Desinfizieren der Dosen (B) ausbilden.

2. Desinfektionsvorrichtung (1) nach Anspruch 1, **dadurch gekennzeichnet, dass** als Behandlungsabschnitt (10, 10', 20, 20', 30, 40, 50) zumindest ein Beaufschlagungsabschnitt (10, 10') für eine Beaufschlagung der Dosen (B) mit dem Desinfektionsmittel vorgesehen ist, wobei der zumindest eine Beaufschlagungsabschnitt (10, 10') durch einen Einlaufbereich (3.1) der geneigten Förderstrecke (3) gebildet ist und/oder durch die erste horizontal verlaufende Förderstrecke (4) gebildet ist, wobei die die erste horizontal verlaufende Förderstrecke (4) vor der geneigten Förderstrecke (3) angeordnet ist.

3. Desinfektionsvorrichtung (1) nach Anspruch 1, **dadurch gekennzeichnet, dass** die erste horizontal verlaufende Förderstrecke (4) nach der geneigten Förderstrecke (3) angeordnet ist und einen als Abtropf- und/oder Trocknungsabschnitt (20') ausgebildeten Behandlungsabschnitt (10, 10', 20, 20', 30, 40, 50) bildet.

4. Desinfektionsvorrichtung (1) nach einem der vorangehenden Ansprüche, **dadurch gekennzeichnet, dass** die Fördereinrichtung (2) ferner wenigstens eine in Transportrichtung (TR) vor oder nach der geneigten Förderstrecke (3) angeordnete zweite horizontal verlaufende Förderstrecke aufweist, wobei die zweite horizontal verlaufende Förderstrecke ebenfalls einen Behandlungsabschnitt (10, 10', 20, 20', 30, 40, 50) zur Durchführung von zumindest einem Behandlungsschritt beim Desinfizieren der Dosen (B) ausbildet.

5. Desinfektionsvorrichtung (1) nach einem der vorangehenden Ansprüche, **dadurch gekennzeichnet, dass** die geneigte Förderstrecke (3) zumindest abschnittsweise wenigstens einen als Einwirk- und/oder Aktivierungsabschnitt (40) und/oder einen als Trocknungs- und/oder Aktivierungsabschnitt (50) und/oder einen als Spülabschnitt (30) ausgebildeten Behandlungsabschnitt (10, 10', 20, 30, 30') bildet.

6. Desinfektionsvorrichtung (1) nach Anspruch 2, **dadurch gekennzeichnet, dass** in dem zumindest einen Beaufschlagungsabschnitt (10, 10') eine Beaufschlagungsvorrichtung (6) zum Beaufschlagen der Dosen (B) mit dem Desinfektionsmittel vorgesehen ist.

7. Desinfektionsvorrichtung (1) nach Anspruch 6, **dadurch gekennzeichnet, dass** die Beaufschlagungsvorrichtung (6) eine Sprüh- oder Blasvorrichtung ist, die insbesondere mit einer oder mehreren Düsen ausgestattet ist.

8. Desinfektionsvorrichtung (1) nach einem der vorangehenden Ansprüche, **dadurch gekennzeichnet, dass** ferner eine Heizeinrichtung (5) und/oder eine Spüleinrichtung (7) und/oder eine Trocknungseinrichtung vorgesehen sind.

9. Desinfektionsvorrichtung (1) nach einem der vorangehenden Ansprüche, **dadurch gekennzeichnet, dass** die geneigte Förderstrecke (3) einen Rinser, insbesondere einen Gravitationsrinser, bildet.

10. Desinfektionsvorrichtung (1) nach einem der vorangehenden Ansprüche, **gekennzeichnet durch** eine Hygiene-Einhausung (8), die einen Reinraum oder Sterilraum für die Behandlung der Dosen (B) begrenzt, wobei ferner eine Sterilluftzuführung (9) zur Sterilluftüberlagerung des Reinraums oder Sterilraums vorgesehen ist.

11. Desinfektionsvorrichtung (1) nach einem der vorangehenden Ansprüche, **dadurch gekennzeichnet, dass** in der geneigten Förderstrecke (3) mindestens eine Wendevorrichtung vorgesehen ist, um die Dosen (B) zwischen einer Normalstellung und einer Überkopfstellung zu wenden und/oder dass im Bereich der ersten horizontal verlaufenden Förderstrecke (4) eine Wendevorrichtung, insbesondere ein Dosenwender vorgesehen ist, um die Dosen (B) zwischen einer Normalstellung und einer Überkopfstellung zu wenden.

12. Behandlungsanlage (100) zum Behandeln von Dosen (B) aufweisend eine Desinfektionsvorrichtung (1) gemäß einem der vorangehenden Ansprüche, eine der Desinfektionsvorrichtung (1) nachgeschaltete Füllvorrichtung (101) zum Befüllen der Dosen (B) mit einem flüssigen Füllgut und eine der Füllvorrichtung (101) nachgeschaltete und/oder mit der Füllvorrichtung (101) verblockte Schließvorrichtung (103) zum Verschließen der befüllten Dosen (B).

13. Behandlungsanlage (100) nach Anspruch 12, **dadurch gekennzeichnet, dass** die Schließvorrichtung (103) eine Dosendeckelzuführung (104) mit einer Dosendeckeldesinfektionsvorrichtung (105) umfasst.

14. Behandlungsanlage (100) nach Anspruch 12 oder 13, **dadurch gekennzeichnet, dass** die Behandlungsanlage (100) ferner eine mit der Füllvorrichtung (101) verbundene Kurzzeiterhitzungsanlage (106) zum Sterilisieren des Füllguts vor dessen Zuführung zu der Füllvorrichtung (101) aufweist.

15. Behandlungsanlage (100) nach einem der Ansprüche 12 bis 14, **gekennzeichnet durch** eine Hygiene-Einhausung, in welcher die Füllvorrichtung (101) aufgenommen ist und die unmittelbar an die Desinfektionsvorrichtung (1) anschließt.

16. Behandlungsanlage nach einem der Ansprüche 12 bis 15, **dadurch gekennzeichnet, dass** der Schließvorrichtung (103) ohne Zwischenschaltung einer Pasteurisationsanlage eine Dosen-Verpackungsmaschine und/oder Dosen-Palettiermaschine nachgeschaltet ist.

17. Verfahren zum Desinfizieren von Dosen (B) mit einem Desinfektionsmittel, bei dem die Dosen (B) mittels einer Fördereinrichtung (2) einer Desinfektionsvorrichtung (1) in einer Transportrichtung (TR) ausgehend von einem höheren ersten Höhenniveau (h1) zu einem niedrigeren zweiten Höhenniveau (h2) entlang zumindest einer geneigten Förderstrecke (3) sowie entlang zumindest einer vor oder nach der geneigten Förderstrecke (3) angeordneten ersten horizontal verlaufenden Förderstrecke (4) gefördert werden, wobei die Dosen (B) bei dem Verfahren sowohl in einem durch die erste horizontal verlaufende Förderstrecke (4) gebildeten Behandlungsabschnitt (10, 10', 20, 20', 30, 40, 50) als auch in einem durch die geneigte Förderstrecke (3) gebildeten Behandlungsabschnitt (10, 10', 20, 20', 30, 40, 50) mit jeweils zumindest einem Behandlungsschritt zum Desinfizieren behandelt werden.
